(19) Europäisches Patentamt
European Patent Office
Office européen des brevets

(11) Veröffentlichungsnummer: **0 050 686**
**A1**

(12) # EUROPÄISCHE PATENTANMELDUNG

(21) Anmeldenummer: **80106732.3**

(22) Anmeldetag: **03.11.80**

(51) Int. Cl.³: **C 11 D 3/30**
C 11 D 3/384, C 11 D 1/32
A 61 K 7/08

(30) Priorität: **24.10.80 DE 3040083**

(43) Veröffentlichungstag der Anmeldung:
**05.05.82 Patentblatt 82/18**

(84) Benannte Vertragsstaaten:
**AT BE CH DE FR GB IT LI NL SE**

(71) Anmelder: **Henkel Kommanditgesellschaft auf Aktien**
**-Patentabteilung- Postfach 1100 Henkelstrasse 67**
**D-4000 Düsseldorf 1(DE)**

(72) Erfinder: **Fischer, Herbert, Dr.**
**Kamper Weg 139**
**D-4000 Düsseldorf 12(DE)**

(72) Erfinder: **Stader, Karl Wolfgang**
**Woltmannstrasse 37**
**D-5650 Solingen(DE)**

(72) Erfinder: **Zeidler, Ulrich, Dr.**
**Heinrich-Lersch-Strasse 19**
**D-4000 Düsseldorf 13(DE)**

(54) **Aniontenside mit einem Gehalt an abgebautem Protein, deren Herstellung und Verwendung.**

(57) Anionische Tenside, die als Gegenion zu dem Tensidanion ein Gemisch aus einem Alkanolamin und einem durch Abbau von Eiweißkörpern mittels dieses Alkanolamins gewonnenen Proteinaminolysat enthalten, deren Herstellung und Verwendung sowie diese enthaltende Wasch-, Spül- und Reinigungsmittel sowie kosmetische Zubereitungen.

EP 0 050 686 A1

0050686

HENKEL KGaA
ZR-FE/Patente
dr.gl-sü

Henkelstraße 67
D-4000 Düsseldorf, den

# P a t e n t a n m e l d u n g

## D 6271 EP

"Aniontenside mit einem Gehalt an abgebautem Protein, deren Herstellung und Verwendung"

Gegenstand der Erfindung sind neuartige Tenside auf Basis von grenzflächenaktiven Anionen, insbesondere vom Typ der grenzflächenaktiven Sulfate und Sulfonate, die als Kationen Gemische aus Alkanolaminen und Proteinaminolysaten auf Basis dieser Alkanolamine enthalten. Ein weiterer Gegenstand der Erfindung ist ein einfaches Verfahren zur Herstellung der erfindungsgemäßen Tenside und die Verwendung dieser Tenside in hautverträglichen Wasch- und Reinigungsmitteln sowie diese enthaltende Reinigungsmittel und kosmetische Zubereitungen.

Es ist seit langem bekannt, daß die Hautverträglichkeit von Tensiden und tensidhaltigen Produkten durch Zusatz von modifizierten und wasserlöslich gemachten Eiweißstoffen merklich verbessert werden kann. Hierzu werden Eiweißkörper meist durch Hydrolyse so weit abgebaut, daß sie wasserlöslich und mit den üblichen Bestandteilen tensidhaltiger Formulierungen mischbar sind. Für die Hydrolyse werden zum Beispiel anorganische Säuren oder Alkalien im Überschuß eingesetzt. Die auf diese Weise erhaltenen Hydrolysate müssen neutralisiert werden und enthalten aus diesem Grunde recht hohe Elektrolytgehalte, die beim technischen Einsatz unerwünscht sind oder, zum Beispiel in kosmetischen Präparaten, stören. Eine Reinigung der

...

Proteinhydrolysate durch Abtrennung der Elektrolyte ist aufwendig und teuer. Ein Ausweg wurde zum Beispiel im enzymatischen Abbau von Eiweißstoffen gesucht, da auf diese Weise Hydrolysate mit geringem Elektrolytgehalt entstehen. Nachteil dieses Verfahrens ist die geringe Raum-Zeit-Ausbeute, da man in niedriger Konzentration arbeiten muß; deshalb ist auch dieses Verfahren recht teuer.

Eine weitere Möglichkeit des Eiweißabbaus besteht in der Aminolyse. Auch diese Aufschlußmöglichkeit für Proteine ist seit langem bekannt, und auf diesem Wege erhaltene Eiweiß-Abbauprodukte sind als Additive zur Verbesserung der Hautverträglichkeit von Waschrohstoffen, speziell für den Bereich der Kosmetik im Handel.

Auch Kondensationsprodukte aus Proteinen bzw. Proteinabbauprodukten mit zum Beispiel Fettsäuren sind als Additive für kosmetische Tensidformulierungen bekannt.

Die meisten dieser Produkte sind teuer und zeigen erhebliche anwendungstechnische Mängel, zum Beispiel unbefriedigende Mischbarkeit bzw. Unverträglichkeit mit manchen Tensiden, Beeinträchtigung des Schäumvermögens, der Viskosität, der Farbe und des Geruchs der Formulierungen.

Es bestand daher ein Bedürfnis an preiswerten, einfach zugänglichen Tensid-Protein-Kombinationen, die für die Herstellung von Wasch- und Reinigungsmitteln sowie von kosmetischen Wasch- und Badepräparaten geeignet sind und die beschriebenen Mängel nicht oder in deutlich geringerem Maße aufweisen.

...

Diese Aufgabe wurde erfindungsgemäß dadurch gelöst, daß ein Proteinaminolysat, das durch Eiweißabbau mittels eines Alkanolamins der allgemeinen Formel I

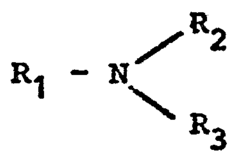

(I)

in welcher $R_1$ eine 2-Hydroxyalkylgruppe mit 2 - 4 Kohlenstoffatomen und $R_2$ und $R_3$ Wasserstoff, Alkylgruppen mit 1 - 4 Kohlenstoffatomen oder 2-Hydroxyalkylgruppen mit 2 - 4 Kohlenstoffatomen bedeuten, zusammen mit einem Überschuß dieses Alkanolamins als Neutralisationsbase für die Tensidanionsäure verwendet wurde.

Die Alkanolamine gemäß der allgemeinen Formel I werden als Neutralisationsbasen für Aniontenside anstelle von Alkalihydroxid seit langem verwendet, da sie zu Tensiden mit günstigerer Hautverträglichkeit führen, als die Alkalihydroxide. Am weitesten verbreitet ist die Verwendung von Monoethanolamin, Diethanolamin und Triethanolamin sowie der homologen Isopropanolamine. Daß sich Alkanolamine als Aufschlußmaterial für Keratin eignen, war ebenfalls schon bekannt, zum Beispiel aus der Deutschen Offenlegungsschrift 2 250 864. Die dort beschriebenen Keratin-Abbauprodukte sind, vor allem wegen des hohen Cystingehaltes und des dadurch bedingten hohen Schwefelgehaltes der Abbauprodukte, farblich und geruchlich sehr unbefriedigend. Neu und von verblüffender Einfachheit und Wirksamkeit ist jedoch die erfindungsgemäße Verwendung der Proteinaminolysate auf Basis von Alkanolamin vorgenannter Formel als Neutralisationsbasen für Aniontenside. Das für die Aminolyse im Überschuß eingesetzte Alkanolamin braucht dabei nicht abgetrennt zu werden, sondern bildet mit der Tensidanionsäure selbst ein wertvolles Aniontensid.

...

Gleichzeitig wird durch dieses Verfahren eine optimale Verträglichkeit zwischen dem Eiweißabbauprodukt und den Aniontensiden erzielt.

Als Tensidanionsäuren kommen alle technisch eingesetzten grenzflächenaktiven Schwefelsäurehalbester und Sulfonsäuren in Frage. Prinzipiell können aber auch andere Anionsäuren, zum Beispiel Fettsäuren, Alkylpolyglycolethercarbonsäuren, Alkyl-, Alkylpolyglycolether- und Alkylarylpolyglycoletherphosphorsäuren usw. verwendet werden. Besonders hervorzuheben sind die Alkylschwefelsäurehalbester mit 8 - 18 Kohlenstoffatomen, die Alkylpolyglycoletherschwefelsäurehalbester mit Alkylgruppen von 8 - 18 Kohlenstoffatomen und 1 - 12 Glycolethergruppen, die Alkylphenolpolyglycolether-Schwefelsäurehalbester mit Alkylgruppen von 8 - 12 Kohlenstoffatomen und 1 - 12 Glycolethergruppen, die Alkylbenzolsulfonsäuren mit Alkylketten von 10 - 14 Kohlenstoffatomen, die alpha-Olefinsulfonsäuren mit 8 - 18 Kohlenstoffatomen, die sekundären Alkansulfonsäuren mit 10 - 18 Kohlenstoffatomen und die Fettsäuremethylester-alphasulfonsäuren auf Basis von Fettsäuren mit 8 - 18 Kohlenstoffatomen.

Als Eiweißkörper, die sich erfindungsgemäß für den Abbau mit Alkanolaminen eignen, können alle pflanzlichen, tierischen, mikrobiellen und synthetischen Proteine verwendet werden. Als Beispiele seien Sojaprotein, Weizengluten, Kollagen, Gelatine, Haut- bzw. Ledereiweiß und Casein genannt, da diese sich wegen des niedrigen Gehaltes an schwefelhaltigen Aminosäuren für das erfindungsgemäße Verfahren besonders gut eignen.

...

Die erfindungsgemäßen neuen Tenside zeichnen sich durch besonders günstige Anwendungseigenschaften aus. Neben der problemlosen Mischbarkeit und Konfektionierbarkeit mit Tensiden aller Art und mit den in flüssigen Wasch- und Reinigungsmitteln sowie kosmetischen Wasch- und Badepräparaten üblichen Hilfsmitteln, vor allem Elektrolyten, Verdickungsmitteln und Schaumboostern, zum Beispiel vom Typ der Fettsäurealkanolamide, Duftstoffen und Konservierungsmitteln zeigen die neuen Tenside selbst ein starkes Schäumvermögen und vor allem eine sehr gute Hautverträglichkeit. Als Maß für die Haut- und Schleimhautverträglichkeit von Aniontensiden kann man unter anderem die Löslichkeit von Proteinen, zum Beispiel von Zein, unter definierten Prüfbedingungen in der Tensidlösung heranziehen, da direkte dermatologische Verträglichkeitsprüfungen der quantitativen Auswertung für Vergleichszwecke nur schwer zugänglich sind (vgl. E. Götte, Proceedings of the IVth International Congress on Surface Active Substances, Brussels 1964, Vol. III, Gordon and Breach Sci. Publ. Inc. New York, 1967).

Auf Grund der günstigen Anwendungseigenschaften sind die erfindungsgemäßen Tenside besonders geeignet zur Herstellung von hautfreundlichen flüssigen Wasch- und Reinigungsmitteln und von kosmetischen Wasch- und Badepräparaten. Hierzu gehören zum Beispiel Geschirrspülmittel, Haushaltsreiniger und Waschmittel für manuelle Anwendung zum Beispiel im Waschbecken, Badezusätze, Duschbäder, Haarshampoos, flüssige Händereinigungsmittel usw. Die erfindungsgemäßen Tenside können in den genannten Produkten ganz oder teilweise an Stelle der bisher üblichen Tenside verwendet werden. Der Verwendung in kosmetischen Präparaten kommt vor allem die helle Farbe und

...

der nur schwach ausgeprägte Geruch der erfindungsgemäßen Tenside entgegen, denn dunkle Farbe und unangenehmer Geruch sind als die typischen Nachteile vieler Proteinderivate bekannt.

Wasch- und Reinigungsmittel, insbesondere kosmetische Zubereitungen mit einem Gehalt an den erfindungsgemäßen Tensiden in Mengen von 10 - 70 Gewichtsprozent der gesamten Zubereitung sind ebenfalls Gegenstand der Erfindung. Solche Produkte können alle in der Praxis üblichen Zusatzmittel, zum Beispiel Verdickungsmittel, Überfettungsmittel, Duftstoffe, Lösungsvermittler, Phosphate usw. enthalten, ohne daß Schwierigkeiten bei der Formulierung oder in der Lagerstabilität auftreten.

Die nachfolgenden Beispiele sollen den Erfindungsgegenstand näher erläutern, ohne ihn jedoch hierauf zu beschränken.

...

## Beispiele

### 1. Herstellung des Ledereiweiß-Monoethanolamin-Abbauproduktes

a) 2 kg Ledereiweiß in Pulverform wurden in 4 l heißem Wasser bei 95 °C 1 Stunde lang vorgequollen. Dann wurden 800 g (13 Mol) Monoethanolamin hinzugefügt und die Mischung 7 Stunden bei 95 °C gerührt. Nach dem Erkalten wurde die Lösung von ungelösten Anteilen (unter 5 %!) abfiltriert und durch Zusatz von 1 % Wasserstoffperoxid-Lösung (30 %ig) gebleicht.

Zur Bestimmung des Gehalts an alpha-Aminogruppen in dem Aminolysat trennt man auf folgende Weise das Proteinderivat vom überschüssigen Monoethanolamin : 50 g des Aminolysats werden zu circa 1 l Isopropanol zugetropft, der gebildete Niederschlag wird abfiltriert, zweimal mit Isopropanol nachgewaschen und dann in Wasser gelöst. Die wäßrige Lösung des Proteinaminolysats wird potentiometrisch titriert.

Das nach 1. a) hergestellte Aminolysat enthielt 0,94 Milliäquivalent alpha-Aminogruppen pro Gramm Substanz, dies entspricht einem mittleren Molekulargewicht von 1060 für das Protein-Aminolysat.

...

2. Herstellung des mit dem Protein-Aminolysat neutralisierten Fettalkoholethersulfats

Die nach Beispiel 1 hergestellte Lösung wurde mit circa 3,5 l Wasser verdünnt. Zu dieser Lösung wurden unter Rühren und Kühlung 3,52 kg eines Lauryl-myristyl-(70:30)-diglycolether-schwefelsäuremonoesters (circa 9,8 Mol) langsam zugegeben. Es wurde ein Produkt mit den folgenden technischen Daten erhalten:

| | |
|---|---|
| Aktivsubstanzgehalt: | circa 45 % |
| Proteinaminolysatgehalt: | circa 17 % |
| pH-Wert: | circa 8,0 |
| Jodfarbzahl: | circa 10 |
| Viskosität (20 °C): | 2 000 m . Pa . s |

Das Produkt hat einen schwachen Eigengeruch.

...

3. Anwendungstechnische Eigenschaften des nach Beispiel 2 hergestellten Tensids - im Vergleich zu Standardprodukten

3. a) Schaumvergleich nach DIN 53902 (Schlagschaumbestimmung

| Produkt | | Tensidkonzentration | | |
|---|---|---|---|---|
| | | 0,5 g/l | 1,0 g/l | 2,0 g/l |
| Prod. aus Beispiel 2 | Schaumvolumen | 610 | 700 | 780 |
| | Zerfall (ml/Min) | 8,0 | 7,5 | 10,5 |
| Gemisch (50/50) aus Prod. aus Beisp. 2 u. Texapon N 25 | Schaumvolumen | 650 | 780 | 850 |
| | Zerfall (ml/Min) | 8,0 | 10,0 | 11,0 |
| Texapon N 25 (Lauryl-myristyl-di-glycol-ethersulfat Na-Salz) | Schaumvolumen | 800 | 880 | 880 |
| | Zerfall (ml/Min) | 12,5 | 13,0 | 16,5 |

3. b) Hautverträglichkeit, gemessen am Lösevermögen für Zein (nach E. Götte, Proceedings of the IVth. Int. Congress on Surf. Act. Subst., Brussels 1964, Vol. III, Gordon and Breach Sci. Publ. Inc. N.Y. 1967)

...

| Produkt | Konzentration | Zein-Zahl+) (mgN/100 ml) |
|---|---|---|
| Produkt nach Beispiel 2 | 20 %ig | 125 |
| Fettalkohol $C_{12-14}$ + 2 EO-sulfat, Na-Salz | 20 %ig | 288 |
| Laurylsulfat, Na-Salz | 20 %ig | 535 |

+) Die Zein-Zahl gibt die unter Testbedingungen im Tensid gelöste Menge Zein-Stickstoff an. Je niedriger die Zein-Zahl, desto besser ist die Hautverträglichkeit zu bewerten.

4. Beispiele für Reinigungsmittel mit einem Gehalt an erfindungsgemäßem Tensid nach Beispiel 2

a) Reinigungsmittel

20 % Produkt nach Beispiel 2
25 % Trikaliumorthophosphat
0,1 % Parfümöl
Rest Wasser

Aussehen: Klare,gelbliche,niedrigviskose Flüssigkeit

b) Schaumbad

35 % Produkt nach Beispiel 2
5 % Kokosfettsäurediethanolamid
3 % Parfümöl
57 % Wasser

Aussehen: Klare, gelbliche, viskose Flüssigkeit.

...

c) Duschbad

30 % Produkt nach Beispiel 2
3 % Kokosfettsäuremonoethanolamid
5 % Kokosfettsäurediethanolamid
2 % Parfümöl
60 % Wasser

Aussehen: Klare, gelbliche, hochviskose Flüssigkeit

d) Baby-Schaumbad

60 % Produkt nach Beispiel 2
15 % Capryl-/Caprinsäure-Triglycerid
3 % Kamillenextrakt
1 % Parfümöl
21 % Wasser

Aussehen: Klare, gelbe, viskose Flüssigkeit

e) Haar-Shampoo

20 % Produkt nach Beispiel 2
6 % Kokosfettsäuremonoethanolamid
2 % Parfümöl
72 % Wasser

Aussehen: Klare, gelbliche, viskose Flüssigkeit

...

Patentansprüche:

1. Anionische Tenside, dadurch gekennzeichnet, daß als Gegenion zu dem Tensidanion ein Gemisch aus einem Alkanolamin der allgemeinen Formel I

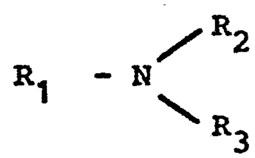

(I)

in welcher $R_1$ eine 2-Hydroxyalkylgruppe mit 2 - 4 Kohlenstoffatomen und $R_2$ und $R_3$ Wasserstoff, Alkylgruppen mit 1 - 4 Kohlenstoffatomen oder 2-Hydroxyalkylgruppen mit 2 - 4 Kohlenstoffatomen bedeuten, und einem durch Aminolyse von Eiweißkörpern mittels dieses Alkanolamins gewonnenes Proteinabbauprodukt mit einem mittleren Molekulargewicht von 500 - 5 000 verwendet wird.

2. Tenside nach Anspruch 1, dadurch gekennzeichnet, daß als Aniontensid ein Alkylsulfat mit 8 - 18 Kohlenstoffatomen, ein Alkylpolyglycolethersulfat mit einer Alkylgruppe von 8 - 18 Kohlenstoffatomen und mit 1 - 12 Glycolethergruppen, ein Alkansulfonat mit 8 - 18 Kohlenstoffatomen, ein alpha-Olefinsulfonat mit 8 - 18 Kohlenstoffatomen oder ein Fettsäuremethylester-alpha-sulfonat auf Basis einer Fettsäure mit 8 - 18 Kohlenstoffatomen verwendet wird.

3. Tenside nach Anspruch 1 und 2, dadurch gekennzeichnet, daß als Eiweißkörper Kasein, Kollagen, Haut- oder Ledereiweiß, Gelatine, Sojaprotein oder Weizengluten verwendet wird.

...

4. Verfahren zur Herstellung von Tensiden nach Anspruch 1 - 3, dadurch gekennzeichnet, daß man Eiweißkörper mit wäßrigen Lösungen von Alkanolaminen der Formel I im Überschuß zur Reaktion bringt und die, gegebenenfalls von ungelösten Anteilen befreite basische Lösung des Proteinaminolysats zur Neutralisation von Tensidanionsäure verwendet.

5. Verwendung der Tenside nach Anspruch 1 - 3 allein oder im Gemisch mit anderen Tensiden zur Herstellung von Wasch-, Spül- und Reinigungsmitteln, insbesondere zur Herstellung von hautverträglichen kosmetischen Zubereitungen.

6. Hautfreundliche Wasch-, Spül- und Reinigungsmittel, insbesondere kosmetische Badepräparate, Shampoos und Körperreinigungsmittel mit einem Gehalt an Tensiden nach Anspruch 1 - 3 in einer Menge von 10 - 70 Gewichtsprozent, bezogen auf das gesamte Mittel.

Patentanmeldung D 6271 AT

HENKEL KGaA
ZR-FE/Patente
9.6.1981
Dr. JG/Po

## Patentansprüche für Österreich

1. Verfahren zur Herstellung von anionischen Tensiden, dadurch gekennzeichnet, daß zur Neutralisation der Tensidanionsäuren ein Gemisch aus einem Alkanolamin der allgemeinen Formel I

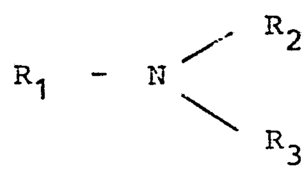

(I)

in welcher $R_1$ eine 2-Hydroxyalkylgruppe mit 2 - 4 Kohlenstoffatomen und $R_2$ und $R_3$ Wasserstoff, Alkylgruppen mit 1 - 4 Kohlenstoffatomen oder 2-Hydroxyalkylgruppen mit 2 - 4 Kohlenstoffatomen bedeuten, und einem durch Aminolyse von Eiweißkörpern mittels dieses Alkanolamins gewonnenen Proteinabbauprodukt mit einem mittleren Molekulargewicht von 500 - 5000 verwendet wird.

2. Verfahren zur Herstellung von anionischen Tensiden nach Anspruch 1, dadurch gekennzeichnet, daß als Tensidanionsäure ein Alkysulfat mit 8 - 18 Kohlenstoffatomen, ein Alkylpolyglycolethersulfat mit einer Alkylgruppe von 8 - 18 Kohlenstoffatomen und mit 1 - 12 Glycolethergruppen, ein Alkansulfonat mit 8 - 18 Kohlenstoffatomen, ein alpha-Olefinsulfonat mit 8 - 18 Kohlenstoffatomen oder ein Fettsäuremethylester-alpha-sulfonat auf Basis einer Fettsäure mit 8 - 18 Kohlenstoffatomen verwendet wird.

...

3. Verfahren zur Herstellung von anionischen Tensiden nach Anspruch 1 und 2, dadurch gekennzeichnet, daß als Eiweißkörper Kasein, Kollagen, Haut- oder Ledereiweiß, Gelatine, Sojaprotein oder Weizengluten verwendet wird.

4. Verfahren zur Herstellung von Tensiden nach Anspruch 1 - 3, dadurch gekennzeichnet, daß man Eiweißkörper mit wäßrigen Lösungen von Alkanolaminen der Formel I im Überschuß zur Reaktion bringt und die, gegebenenfalls von ungelösten Anteilen befreite basische Lösung des Proteinaminolysats zur Neutralisation von Tensidanionsäure verwendet.

5. Verwendung der Tenside, hergestellt nach Anspruch 1 - 3, allein oder im Gemisch mit anderen Tensiden zur Herstellung von Wasch-, Spül- und Reinigungsmitteln, insbesondere zur Herstellung hautverträglichen kosmetischen Zubereitungen.

6. Hautfreundliche Wasch-, Spül- und Reinigungsmittel, insbesondere kosmetische Badepräparate, Shampoos und Körperreinigungsmittel mit einem Gehalt an Tensiden, hergestellt nach Anspruch 1 - 3, in einer Menge von 10 - 70 Gewichtsprozent, bezogen auf das gesamte Mittel.

0050586

Europäisches Patentamt

# EUROPÄISCHER RECHERCHENBERICHT

Nummer der Anmeldung

EP 80 10 6732.3

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | DE - A1 - 2 434 063 (PROCTER & GAMBLE CO.)<br>* Ansprüche 1, 11, 12, 22; Seite 1, Absatz 1; Seite 37, Beispiele 22 bis 28 *<br>& US - A - 4 115 548<br>& US - A - 4 195 077<br>-- | 1-3, 5,6 | C 11 D 3/30<br>C 11 D 3/384<br>C 11 D 1/32<br>A 61 K 7/08 |
| D | DE - A - 2 250 864 (ATHLON CORP.)<br>& GB - A - 1 374 884<br>-- | | |
| A | DE - A1 - 2 544 778 (PROCTER & GAMBLE CO.)<br>-- | | RECHERCHIERTE SACHGEBIETE (Int. Cl.³) |
| A | DE - A - 2 252 281 (KYOWA HAKKO KOGYO K.K.)<br>-- | | A 61 K 7/00<br>C 11 D 1/00<br>C 11 D 3/00 |
| A | DE - A1 - 2 712 228 (G. LEIDERER)<br>---- | | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 26-08-1981 | SCHULTZE |